(19) Europäisches Patentamt
European Patent Office
Office européen des brevets

(11) Publication number: **0 366 483 A2**

# (12) EUROPEAN PATENT APPLICATION

(21) Application number: 89311101.3

(51) Int. Cl.⁵: C08G 65/32

(22) Date of filing: 27.10.89

(30) Priority: 28.10.88 US 263719

(43) Date of publication of application: 02.05.90 Bulletin 90/18

(84) Designated Contracting States: CH DE FR GB IT LI NL

(71) Applicant: MINNESOTA MINING AND MANUFACTURING COMPANY
3M Center, P.O. Box 33427
Saint Paul, MN 55133- 3427(US)

(72) Inventor: Guenthner, Richard A. c/o Minnesota Mining and Manufacturing Company 2501 Hudson Road
St. Paul Minnesota 55133-3427(US)
Inventor: Gutman, Gustav c/o Minnesota Mining and Manufacturing Company 2501 Hudson Road
St. Paul Minnesota 55133-3427(US)

(74) Representative: Baillie, Iain Cameron et al
c/o Ladas & Parry Isartorplatz 5
D-8000 München 2(DE)

## (54) Fluoroaliphatic sulfonamido poly(oxyalkylene) compounds.

(57) Fluoroaliphaticsulfonamido derivatives of alkylaryloxy poly(oxyalkylene) amines are described which are useful as antistatic agents. For example, they are useful in imparting antistatic characteristics to plastic films and the like for packaging electronic components.

EP 0 366 483 A2

# FLUOROALIPHATIC SULFONAMIDO POLY(OXYALKYLENE) COMPOUNDS

This invention relates to derivatives of alkylaryloxy poly(oxyalkylene) amines. More particularly, this invention relates to sulfonamido derivatives of these amines. Even more particularly, this invention relates to fluoroaliphaticsulfonamido derivatives of these amines having utility as antistatic agents.

A number of fluoroaliphatic sulfonamide compounds, oligomers and polymers containing poly(oxyalkylene) groups or chains are known.

For example, U.S. Patent No. 2,915,554 (Ahlbrecht) discloses the preparation of non-ionic surfactant derivatives, N(polyoxa-alkyl)-perfluoroalkanesulfonamides, which can be represented by the general formula, $R_fSO_2N(R)(R^1)$ where $R_f$ is a perfluoroalkyl group having from 4 to 12 carbon atoms, R is a member of the group consisting of H, lower alkyl and $R'$; and $R'$ is a polyoxa-alkyl group having the formula $(CH_2)_m$-$(OCH_2CHR'')_nOR''$ in which m is 2 or 3, n is a number from 2 to about 20, and each $R''$ is hydrogen or methyl radical. Specific compounds include $C_8F_{17}SO_2NH(CH_3)(CH_2CH_2O)_3OCH_3$ and $C_8F_{17}SO_2N(CH_3)$-$C_2H_4(OCH_2CH_2)_{13}OH$.

Other references to fluoroaliphatic sulfonamide poly(oxyalkylene) compositions include U. S. Patent Nos. 4,289,892 (Soch) and 4,710,449 (Lewis et al.). None of these references disclose the fluoroaliphatic sulfonamide compositions of this invention.

The patent application FR 2025688 lists a number of fluorochemical surfactants, including anionic, amphoteric and non-ionic materials, reported to be useful as antistatic agents in photographic emulsions. One of the non-ionic compounds listed is $C_8F_{17}SO_2N(C_2H_5)(CH_2CH_2O)_{14}H$. However, the compounds of the present invention are not disclosed.

There have not heretofore been provided polyether compounds of the type described herein exhibiting desirable antistatic characteristics.

In accordance with the present invention there are provided highly fluorinated alkanesulfonamido derivatives of alkylaryloxy poly(oxyalkylene) amines. These compounds are of the formula

$$R_fSO_2\ \overset{\overset{R}{|}}{N}-(R''O)_y-(R'''O)_n-R'$$

where y and n are each in the range of 0 to 100; wherein y + n is at least 2; wherein R is hydrogen, alkyl, aryl, aralkyl, aminoalkyl, or hydroxyalkyl; wherein $R''$ and $R'''$ are selected from ethylene and propylene; and wherein $R'$ is aryl, aralkyl or alkaryl.

These compounds are useful as antistatic agents in various applications. For example, these compounds provide desirable and beneficial antistatic properties to plastic films. In one embodiment such films or sheet materials may be used for packaging electronic components, and they are sufficiently transparent to enable visual identification of components through the sheet material.

The compounds of the invention are represented by the general formula

$$R_fSO_2\ \overset{\overset{R}{|}}{N}-(R''O)_y-(R'''O)_n-R'$$

where y and n are each in the range of 0 to 100; wherein y + n is at least 2; wherein R is hydrogen, alkyl, aryl, aralkyl, aminoalkyl, or hydroxyalkyl; where $R''$ and $R'''$ are selected from ethylene and propylene; and wherein $R'$ is aryl, aralkyl or alkaryl. Where y and n units are both present in the compound they may be either randomly distributed throughout or they may be present in blocks of y and n units.

The fluoroaliphatic radical, $R_f$, is a fluorinated, stable, inert, non-polar, preferably saturated, monovalent moiety which, if sufficiently large (e.g., 5 or more carbons), is both hydrophobic and oleophobic. It can be straight chain, branched chain, or, if sufficiently large, cyclic, or combinations thereof, such as alkylcycloaliphatic radicals. The skeletal chain in the fluoroaliphatic radical can include catenary divalent oxygen atoms and/or trivalent nitrogen atoms bonded only to carbon atoms. Generally $R_f$ will have 1 to 30 carbon atoms, preferably 4 to about 12 carbon atoms, and will contain about 40 to 78 weight percent, preferably 50 to 78 weight percent, carbon-bound fluorine. The terminal portion of the $R_f$ group has preferably at least one trifluoromethyl group, and preferably has a terminal group of at least three fully fluorinated carbon atoms, e.g., $CF_3CF_2CF_2$-. The preferred $R_f$ groups are fully or substantially fluorinated, as in the case where $R_f$ is perfluoroalkyl, $C_mF_{2m+1}$-.

More preferably the polyether compound is of the formula

$$C_mF_{2m+1}SO_2\ \overset{\overset{R}{|}}{N}-(CH_2CH_2O)_n-R'$$

where m is an integer of 1 to 12; n is in the range of 2 to 20; R is hydrogen, alkyl, aryl, aralkyl, aminoalkyl, or hydroxyalkyl; and $R'$ is aryl, aralkyl or alkaryl.

The aryl groups referred to in the above formula include phenyl, substituted phenyl, polycyclic

structures, heterocyclic rings, and the like. The aralkyl groups include phenyl groups with aliphatic substituents (such as alkyl and substituted alkyl radicals). The aliphatic chain may include occasional oxygen atoms between adjacent carbon atoms.

Specific polyether compounds of the above formulas include those listed below.

Compound Number

(1) 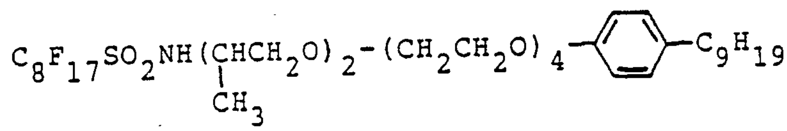

(2) 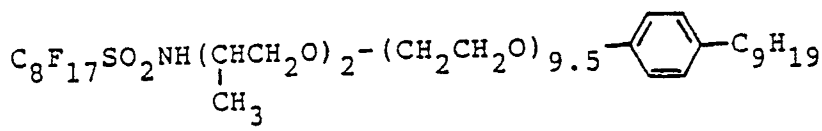

(3) 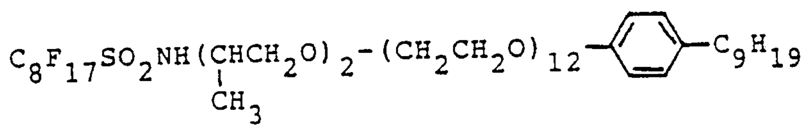

(4) 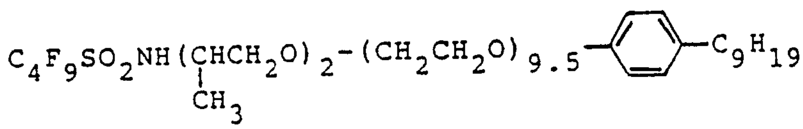

Compounds of the foregoing types may be easily prepared by reacting a fluoroaliphaticsulfonyl fluoride compound with a polyether amine (e.g., of the surfonamine MNPA series which is commercially available from Texaco). Other methods of preparation may also be used, if desired. For example, procedures for preparing fluoroaliphaticsulfonamide compounds which are derivatives of amines are described in U.S. Patent 2,915,554. Some sulfonate salt may also be produced in the reaction, but such salt is not detrimental to the ability of the desired perfluorosulfonamide polyether compound to function as an antistatic compound in this invention.

Compounds of this invention may be advantageously used to impart antistatic characteristics to an antistatic layer on sheet material, described as follows:

(a) self-supporting electrically insulating film having a volume resistivity of at least about $10^{10}$ ohm-centimeters; the film having upper and lower major surfaces;

(b) an electrically conductive layer carried by the upper surface of the film; the conductive layer providing a surface resistivity no greater than $10^4$ ohms per square;

(c) optionally, a tie layer carried by the lower surface of the film; and

(d) an antistatic layer secured to the tie layer, if a tie layer is present, or to the lower major surface of the film if no tie layer is present, the antistatic layer comprising a polymer having dispersed therein a fluoroaliphaticsulfonamide polyether compound of this invention, wherein the antistatic layer provides a surface resistivity in the range of $10^7$ to $10^{14}$ ohms per square.

The sheet material can be easily formed into a strong hermetically sealable envelope which is useful for protecting electronic components (e.g., metal oxide semiconductors). Such an envelope is capable of shielding an electronic component from external electrostatic fields, provides a path to ground for external static electrical charges contacting the envelope, provides a high impedance to prevent electrostatic charges outside the envelope from reaching the electronic component within the envelope, provides for draining of any electrostatic charges at the inner surface of the envelope that may have developed during manufacturing and packaging, restricts development of electrostatic charges due to relative movement between the electronic component and the inner surface of the envelope and provides a high impedance path for the controlled discharge of such electrostatic charges if they do occur, and enables visual identification of the electronic component in the envelope. Also, the envelope does not shed particles which may be considered contaminants.

As used herein, the term "envelope" refers to any complete enclosure formed by one or more sheets of the sheet material which have their edges secured together. The enclosures can be any shape required to enclose an electronic component. Preferably the envelope is formed from only one sheet of the sheet material in order to ensure electrical continuity of the electrically conductive layer over the entire outer

surface of the envelope and continuity of the antistatic layer over the entire inner surface of the envelope.

In the sheet material the insulating film (e.g., polyester) is optionally adhered to the antistatic layer by means of a tie layer (preferably an acrylate polymer). The antistatic layer includes a fluoroaliphaticsulfonamide polyether compound. Conventional antistatic compounds are not effective in the antistatic layer when there is a tie layer present as described herein.

The antistatic layer in the sheet material has improved electrical properties such that it remains conductive at low humidities (i.e., less than 10%). Also the antistatic compounds described herein are non-corrosive and non-contaminating. A tie layer may be included between the antistatic layer and the insulating film without deleterious effect on the desired surface resistivity of the antistatic layer.

In the sheet materials described above, the tie layer and the antistatic layer are preferably co-extruded, after which the tie layer is adhered to the polyester film by passing the polyester film and the tie layer/antistatic layer composite through nip rollers. The tie layer/antistatic layer composite can be passed under ultraviolet lamps while being passed over a heated drum to improve its bond to the polyester film. Alternatively, the tie layer and the antistatic layer can be extruded separately and then pressed together to form a composite which is then adhered to the polyester films.

The tie layer preferably comprises an acrylate polymer such as ethylene-acrylic acid copolymers or ethylene/methacrylic acid copolymers or other acrylate polymers which are tacky and which have a volume resistivity of at least $10^{10}$ ohm-centimeters.

The antistatic layer preferably comprises low density polyethylene (e.g., 1550P from Eastman Kodak) and 0.05 to 10% by weight (more preferably 0.15 to 1%) of a fluoroaliphaticsulfonamide polyether compound. A linear low density polyethylene works very well in the antistatic layer because it has very good heat-sealing properties. Optionally there may also be included up to about 2% by weight of a surfactant comprising a non-ionic polyether compound (e.g., polyethylene oxide or polypropylene oxide derivatives). The surfactant is helpful because it assists in reducing the tribocharging characteristics of the antistatic layer. Tribocharging can result in build-up of static charges in the antistatic layer due to frictional forces (e.g., as occurring when an object is rubbed against the antistatic layer). Two common types of useful surfactants are Tergitol NP-10 or Igepal 530 (a nonylphenol ethyoxylate) and Carbowax 400 (a polyethylene glycol).

It has also been noted that the use of the fluoroaliphaticsulfonamide polyether compounds described herein in the antistatic layer reduces the coefficient of friction of such layer. This is helpful when working with envelopes made of the sheet material.

It is also possible to emboss the sheet material of the invention. This makes it easier to handle when it is used in envelopes for packaging electronic components.

There may also optionally be included in the antistatic layer a small amount of lithium fluoroaliphaticsulfonate salt which is useful in increasing antistatic or conductive properties of a plastic film to which the antistatic layer is applied, especially at low humidity (e.g., less than about 15% relative humidity). When using a water soluble or long chain fluoroaliphaticsulfonamide polyether compound in the antistatic layer of a film construction, the presence of a lithium perfluorosulfonate salt can improve seam strength of packages made from such film.

The lithium fluoroaliphaticsulfonate salt which is useful herein is of the formula

$R_fSO_3Li$

wherein $R_f$ is a fluoroaliphatic radical as described above having from 1 to about 30 carbon atoms. Lithium disulfonate salts may also be used, e.g., $LiO_3S\text{-}C_nF_{2n}\text{-}SO_3Li$, where n is 1 to about 30.

The amount of lithium salt which may be included in the antistatic layer may vary (e.g., from about 0.01 to 1% by weight based on the weight of the antistatic layer).

The invention is further illustrated by means of the following examples wherein the term parts refers to parts by weight unless otherwise indicated.

## Examples 1-12

Various sheet materials are prepared comprising a polyester film, a polyethylene layer, and an antistatic compound contained in the polyethylene layer. The polyethylene used was either low density (LD) or linear low density (LL). A tie layer, if used, was an acrylate polymer identified in the following Table I as CXA-3101 (an ethylene based acrylate polymer commercially available from Dupont); EAA-459, EAA-435, EAA-3440 (ethylene/acrylic acid copolymers commercially available from DOW); or EMA-2207 (ethylene/methacrylic acid copolymer commercially available from DOW).

The resistivity (ohm/square) of the antistatic layer at 10% relative humidity and 50% relative humidity are shown in the table. The type of antistatic compound is also shown in the table.

TABLE I

| | | | | Resistivity (ohm/square) | |
|---|---|---|---|---|---|
| Example | · Antistat | % | Tie Layer | 50% R.H. | 10% R.H. |
| 1 | Varstat K22 | 0.14 | None | $3.8 X 10^{11}$ | $6 X 10^{12}$ |
| 2 | " | " | CXA-3101 | $>10^{15}$ | $>10^{15}$ |
| 3 | " | " | EAA 459 | " | " |
| 4 | " | " | EAA 435 | " | " |
| 5 | Kemamine 650 | " | None | $3.7 X 10^{11}$ | $6 X 10^{12}$ |
| 6 | " | " | CXA-3101 | $8.9 X 10^{13}$ | $>10^{15}$ |
| 7 | " | " | EAA-459 | $>10^{15}$ | " |
| 8 | " | " | EAA-435 | " | " |
| 9 | Verstat K22 | " | None | $8.9 X 10^{10}$ | $7.4 X 10^{12}$ |
| 10 | " | " | EMA-2207 | $7.4 X 10^{12}$ | $>10^{15}$ |
| 11 | Compound 2 | " | " | $4.6 X 10^{11}$ | $1.5 X 10^{12}$ |
| 12 | Compound 3 | " | " | $4.0 X 10^{11}$ | $1.1 X 10^{12}$ |

In the foregoing examples, Varstat K22 is a commercially-available antistatic compound from Sherex and is identified as N,N-Bis(2-hydroxyethyl)alkyl amine (an ethoxylated cocoamine). Kemamine 650 is also a commercially-available antistatic compound available from Humko Chemical and is chemically similar to Varstat K22.

The foregoing data illustrates that when a tie layer is used to secure the polyethylene to the polyester film the commercially-available antistatic agents are not effective at low humidity. On the other hand, the perfluorosulfonamide polyether compounds are very effective as antistatic agents even at low humidity.

## Example 13

A perfluorosulfonamide polyether compound having the formula of compound 2 above is prepared using the following procedure.

A polyether amine (100 grams) (MNPA-750, commercially available from Texaco), triethylamine (20 grams), and isopropyl ether (100 grams) are dried over potassium hydroxide and then combined in a one-liter flask fitted with a mechanical stirrer and gas inlet. Perfluorooctanesulfonyl fluoride (72 grams; commercially available from 3M as FX-8) is then added to the flask and the mixture is heated at reflux under a nitrogen blanket for 3 hours. The mixture is then cooled and neutralized with 50 mL of 50% concentrated hydrochloric acid.

The reaction mixture is then combined with an equal portion of chloroform, and the chloroform organic layer is washed three times with deionized water. The organic layer is separated and dried over anhydrous magnesium sulfate, filtered, and then the chloroform is removed under vacuum. The product (133 grams) is collected as an amber syrup.

Although some sulfonate salt may also be produced in the reaction, the presence of even major amounts of sulfonate salt is not detrimental to the ability of the desired perfluorosulfonamide polyether compound to function as an antistatic compound in the film constructions described herein. Preparing the perfluorosulfonamide polyether compounds under anhydrous conditions improves the yield of the desired compound. Other compounds of the invention may be prepared in similar fashion using other polyether amines and other perfluoroalkane sulfonyl fluoride compounds.

## Claims

1. A compound of formula

$$R_fSO_2\overset{\overset{R}{|}}{N}-(R''O)_y-(R'''O)_n-R'$$

where y and n are each in the range of 0 to 100; wherein y + n is at least 2; wherein R is hydrogen, alkyl, aryl, aralkyl, aminoalkyl, or hydroxyalkyl; wherein R″ and R‴ are independently selected from ethylene and propylene; and wherein R′ is aryl, aralkyl or alkaryl.

2. A compound in accordance with claim 1, wherein the $R_f$ group comprises a perfluoroalkyl group containing 1 to about 20 carbons.

3. A compound according to claim 2 of the formula

$$C_mF_{2m+1}SO_2\overset{\overset{R}{|}}{N}-(\underset{\underset{CH_3}{|}}{CH}CH_2O)_y-(CH_2CH_2O)_n-R'$$

where m is an integer of 1 to about 30, and y, n, R and R′ are as defined in claim 1.

4. A compound in accordance with claim 3, wherein m is an integer of 1 to 12.

5. A compound in accordance with any one of claims 1 to 4, wherein n is in the range of 2 to 20 and y is in the range of 0 to 40.

6. A compound in accordance with claim 5, wherein y is 0.

7. A compound in accordance with either of claims 5 and 6, wherein R is hydrogen.

8. A compound in accordance with any one of claims 1 to 7, wherein R′ comprises aralkyl.

9. A compound in accordance with claim 8, wherein R′ is

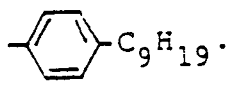

10. A compound in accordance with claim 7, wherein R′ is aryl.